# EUROPEAN PATENT SPECIFICATION

(11) **EP 3 752 047 B1**
(45) Date of publication and mention of the grant of the patent: **06.12.2023**
(21) Application number: 19704335.9
(22) Date of filing: 13.02.2019
(51) Int. Cl.: A61B 5/00, A61B 5/02, A61B 5/087, A61B 5/026, A61B 5/024, A61B 5/0205

(54) **USE OF APPARATUS FOR THE CARDIOVASCULAR ASSESSMENT OF A SUBJECT IN NEED THEREOF**
VERWENDUNG EINER VORRICHTUNG ZUR KARDIOVASKULÄREN BEURTEILUNG EINER PERSON
UTILISATION D'UN APPAREIL D'ÉVALUATION CARDIOVASCULAIRE D'UN SUJET

(30) Priority: 13.02.2018 EP 18382080
(43) Date of publication of application: 23.12.2020
(73) Proprietor: Consorcio Centro de Investigación Biomédica en Red, 28029 Madrid (ES); Asociación Centro de Investigación Cooperativa en Biomateriales - CIC biomaGUNE, 20014 San Sebastián (ES); Fundación Instituto Investigación Sanitaria Fundación Jiménez Díaz, 28040 Madrid (ES)
(72) Inventor: SANTOS OVIEDO, Arnoldo de Jesús, 28029 Madrid (ES); RUIZ-CABELLO OSUNA, Jesús María, 20014 San Sebastián (ES)
(74) Representative: Hoffmann Eitle
(86) International application number: PCT/EP2019/053586
(87) International publication number: WO 2019/158604

(56) References cited:
- EP-A1- 2 198 776
- US-A1- 2008 119 749
- TZENG Y C ET AL: "Paradoxical respiratory sinus arrhythmia in the anesthetized rat", AUTONOMIC NEUROSCIENCE: BASIC AND CLINICAL, ELSEVIER, AMSTERDAM, NL, vol. 118, no. 1-2, 31 March 2005 (2005-03-31), pages 25-31, XP027679965, ISSN: 1566-0702 [retrieved on 2005-03-31]
- JURAK PAVEL ET AL: "Respiratory induced heart rate variability during slow mechanical ventilation", WIENER KLINISCHE WOCHENSCHRIFT, NEW YORK, NY, US, vol. 129, no. 7, 24 February 2017 (2017-02-24), pages 251-258, XP036216123, ISSN: 0043-5325, DOI: 10.1007/S00508-017-1176-0 [retrieved on 2017-02-24]

## Description

### Technical effect

The invention relates to a use of an apparatus by which the cardiovascular status of patients, who are optionally mechanically ventilated, can be assessed by analyzing hemodynamic signals reordered according to the respiratory cycle time at which the heart beat occurs.

### Background of the invention

In recent years two important aspects have demonstrated worsening outcomes in acute respiratory distress syndrome (ARDS): 1) the development of pulmonary artery (PA) and right ventricle (RV) dysfunction, and 2) the use of high driving pressure (plateau-PEEP) during mechanical ventilation (MV). In consequences, suiting protective MV strategy to avoid these phenomena appears necessary to decrease mortality and morbidity in ARDS patients.

Changes in lung volume and pressure affect pulmonary vascular mechanics (PVM). Also it has been demonstrated that ARDS worsen PVM. However the effect of tidal breathing on PVM and if this effect is different during MV in ARDS have been less studied. The last can be particularly relevant as a link between the use of high volumes or driving pressure during MV and tidal worsening of PVM could be found. Such a link could be important from a physiopathology point of view and also for clinical outcomes as pulmonary circulation could be especially sensitive to tidal cycle deleterious effect in a situation of heterogeneity as observed in ARDS.

Cyclic lung volume and pleural pressure change during respiration have been classically considered a problem for PVM evaluation. In this regard, measurements during the last portion of exhalation are recommended. Although this can work as representative of a particular situation, it does not account for real changes that can happen during the respiratory cycle. Such respiratory changes can be particularly important during MV and especially in ARDS as the pulmonary vascular and RV function can be more susceptible to be affected by the lung condition. In the present invention, we demonstrate that lung condition can modify PVM for changes occurring at the respiratory cycle time scale. These observed changes can be explained by the described respiratory effects on preload and afterload: decrease in venous return, increase in the size of extra alveolar capillaries and decrease in the size of the alveolar capillaries.

In this sense, some approaches have been proposed for evaluating PVM during breathing.

However, almost no description about the changes in these effects caused by ARDS is found. One of these proposed methods of evaluation is described in Lieber BB, Li Z, Grant BJ. Beat-by-beat changes of viscoelastic and inertial properties of the pulmonary arteries. J Appl Physiol (1985). Jun 1994;76(6):2348-2355. In this publication, Lieber et al described the change in PVM across the respiratory cycle, however, as far as we can understand the binning on the Lieber et al's analysis is limited to the heart/respiratory rate ratio and they show an increase in PA compliance during inspiration in clear contrast to the results shown in the present invention.

There is thus still a need for a methodology which predicts, among other things, whether the application of MV, and in particular of high driving pressure, might lead to the development pulmonary artery (PA) and right ventricle (RV) dysfunction in ARDS.

TZENG Y C ET AL: "Paradoxical respiratory sinus arrhythmia in the anesthetized rat",AUTONOMIC NEUROSCIENCE: BASIC AND CLINICAL, ELSEVIER, AMSTERDAM, NL, vol. 118, no. 1-2, 31 March 2005 (2005-03-31), pages 25-31, XP027679965,ISSN: 1566-0702, discloses plotting of RSA curves.

JURAK PAVEL ET AL: "Respiratory induced heart rate variability during slow mechanical ventilation",WIENER KLINISCHE WOCHENSCHRIFT, NEW YORK, NY, US, vol. 129, no. 7, 24 February 2017 (2017-02-24), pages 251-258, XP036216123,ISSN: 0043-5325, DOI: 10.1007/ S00508-017-1176-0, discloses plotting of several cardiovascular parameters in a waveform display.

### Brief description of the figures

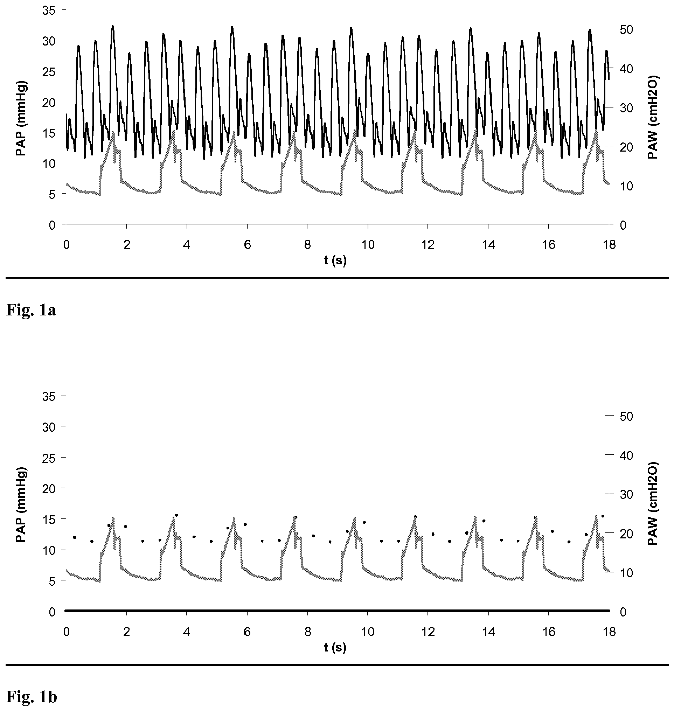

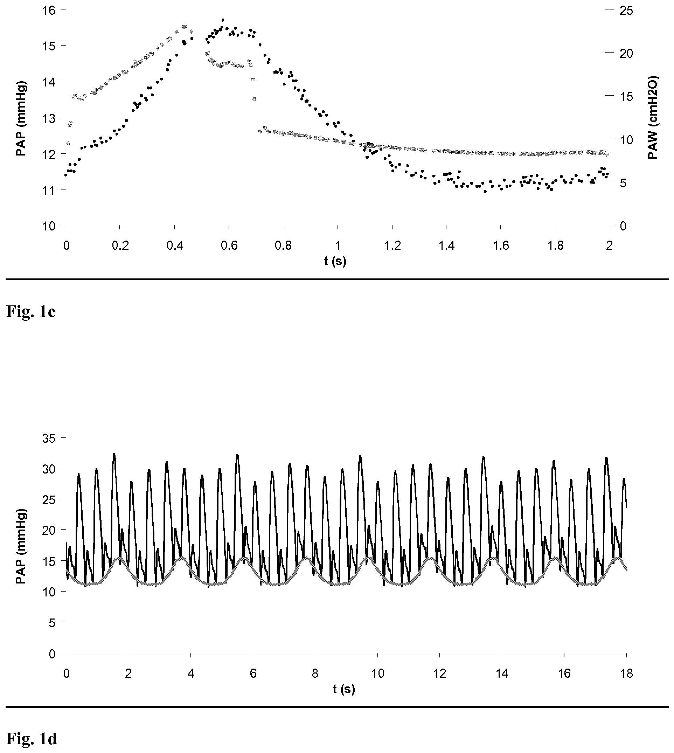

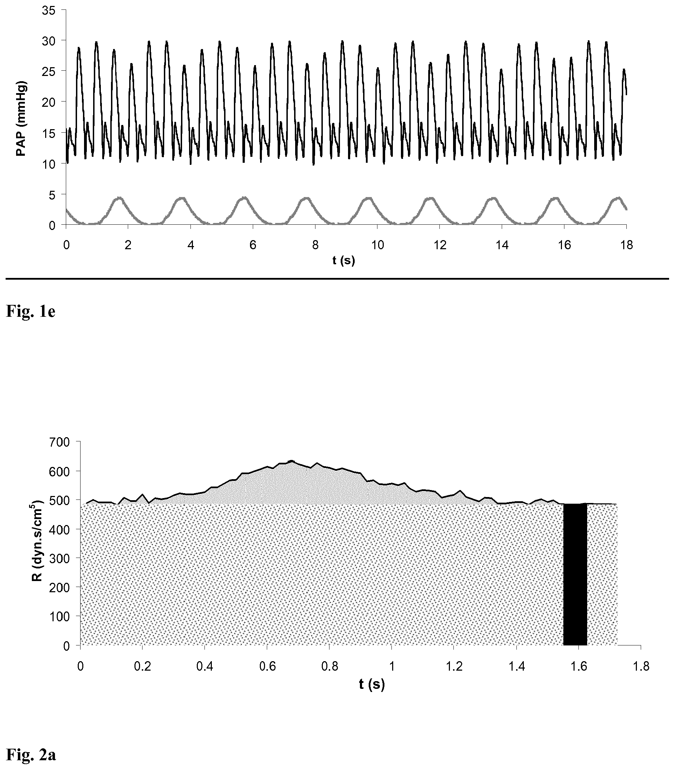

**Fig. 1****.** Correction of the airway pressure effect on pulmonary artery pressure. 1a) Change on PAP provoked by respiration. Black line = PPA pressure signal; Gray line airway pressure signal. 1b) Diastolic pressure (dotted points) and airway pressure across the time. 1c) Reordering of minimum PA pressure (black) at the start of the pressure period and airway pressure measured at the same time and airway pressure (gray). 1d) Built function (gray) for correction of the airway pressure effect on pulmonary artery pressure (black) before escalation. 1e) Corrected Pulmonary artery pressure (black) and the scaled built function that was extracted from original signal pulmonary atery pressure signal. It ca be note that almost no fluctuation form diastole to diastole can be observed but variability on systolic pressure at respiratory cycle time scale remains.

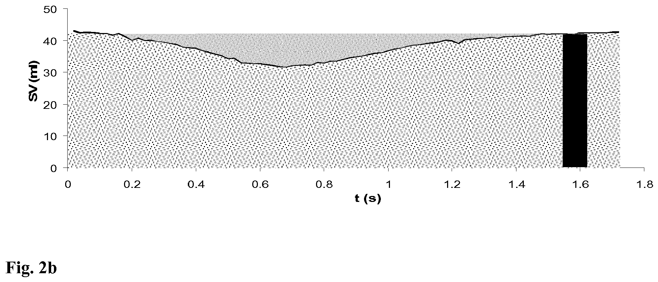

**Fig. 2**. 2a) Example of the method applied to estimate the effect of breathing on pulmonary vascular mechanics. Resistance measured in one animal is shown. The expiratory value is calculated during the time period marked by the black column which leads to the expiration (dotted) area. The effect of inspiration is represented by area above expiration (gray). The total Resistance across the respiration in represented by the sum of inspiratory and expiratory areas. 2b) Example of the stroke volume during the respiration. Fir this variable the expiratory area is bigger than total measured area leading to a negative inspiratory area.

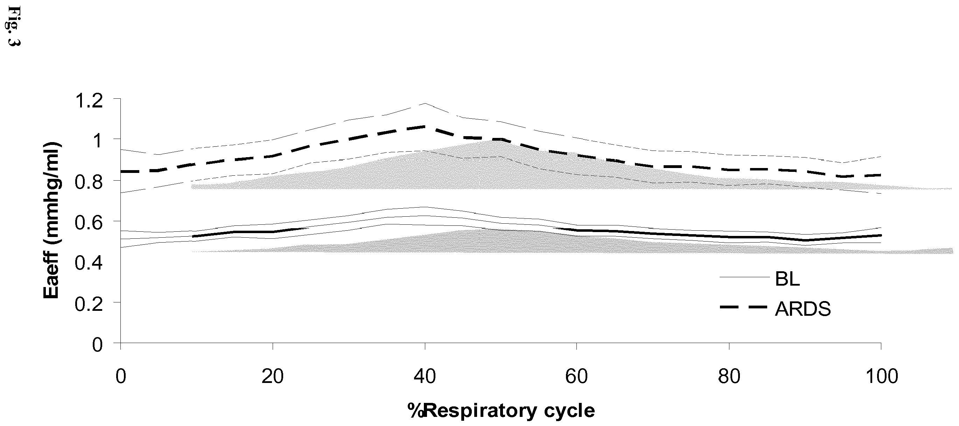

**Fig. 3****.** Curves representing effective arterial elastance (Eaeff) across respiration for the six studied animal at baseline (BL) and (ARDS). The curves resulting form the analysis of each animal were represented as a function of the percent of the respiratory cycle period and then averaged. X axis was binned at 5% intervals. Marginal lines represent the value at the bin ± 2 SEM. Gray area represents the Eaeff during inspiration.

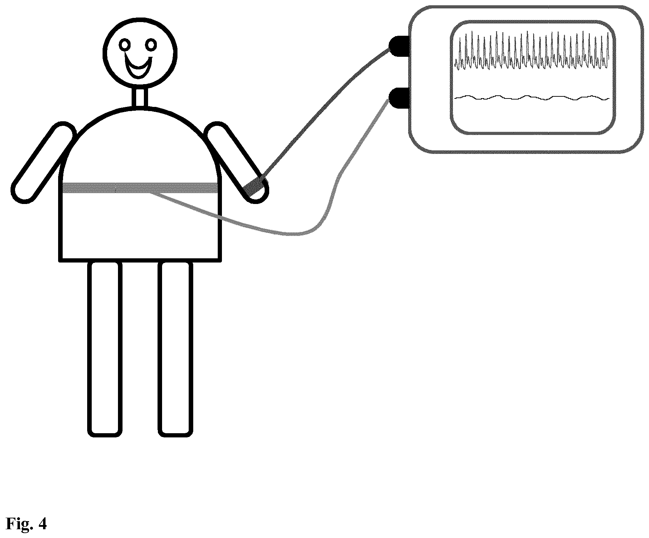

**Fig. 4****.** This figure indicates that the invention requires two signals: a respiratory and a cardiac or hemodynamic signal for obtaining the respiration cycle and the cardiac cycle of the subject.

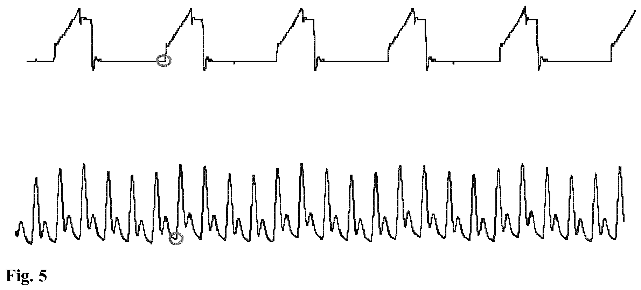

**Fig. 5****.** This figure indicates that in order to practice the use of the present invention, it is deemed necessary to detect, preferably with high precision, the beginning of both the respiratory cycle and the cardiac cycle.

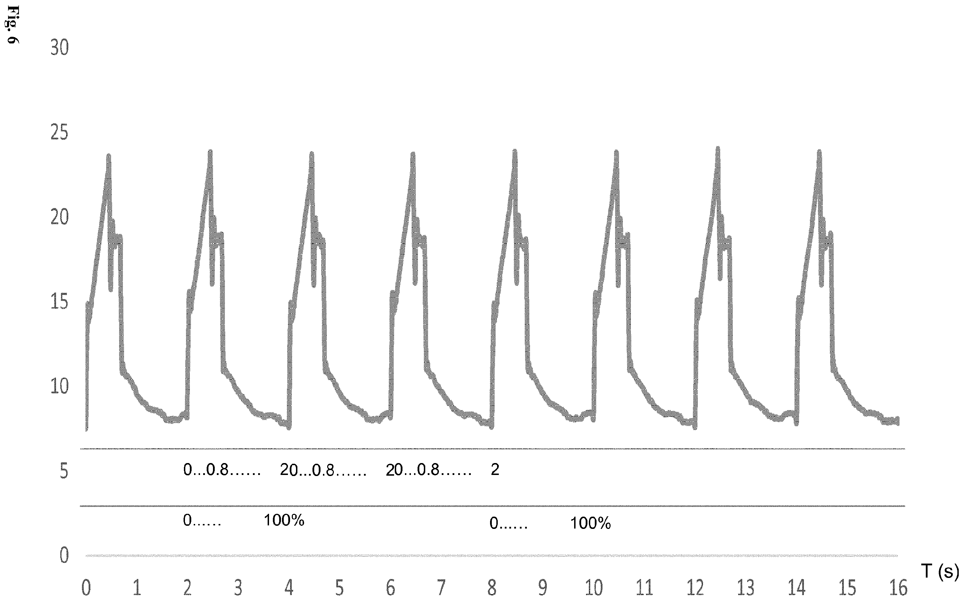

**Fig. 6****.** This figure illustrates that once the start of the respiratory cycle is located, a new definition of the relative times of the respiratory cycle is made with respect to the same cycle. That is to say that the temporalis events begin to be considered relative to the respiratory cycle which begins to be considered as 0 once each respirable cycle begins. Taking into account that each point of the respiratory cycle can be considered in units of time whose maximum will be the duration of the respiratory cycle or in percentages or fractions with respect to each cycle, being the beginning 0 and the maximum 100% or 1. This is considered as such because the cardiac cycles will be reordered based on this relative position. For the reordering of the cardiac cycles it is important to establish the beginning of each cardiac cycle relative to the respiratory cycle so that so that the beginning of each cardiac cycle is place and reordered relative to its position to the respiratory cycle.

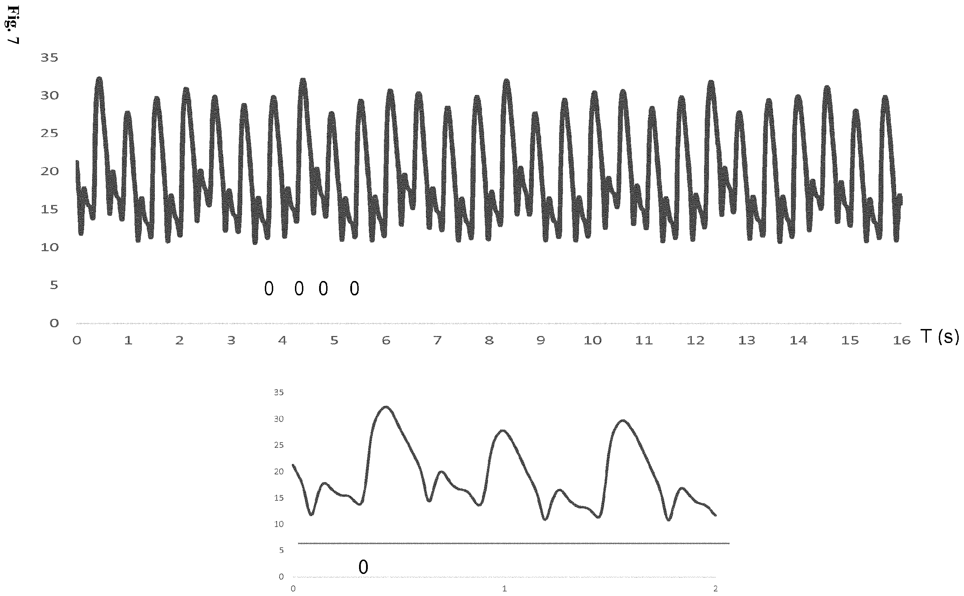

**Fig. 7****.** This figure illustrates that the numbering of the sampling points (corresponding to the hemodynamic or cardiac cycle) is carried out according to the beginning of each respiratory cycle.

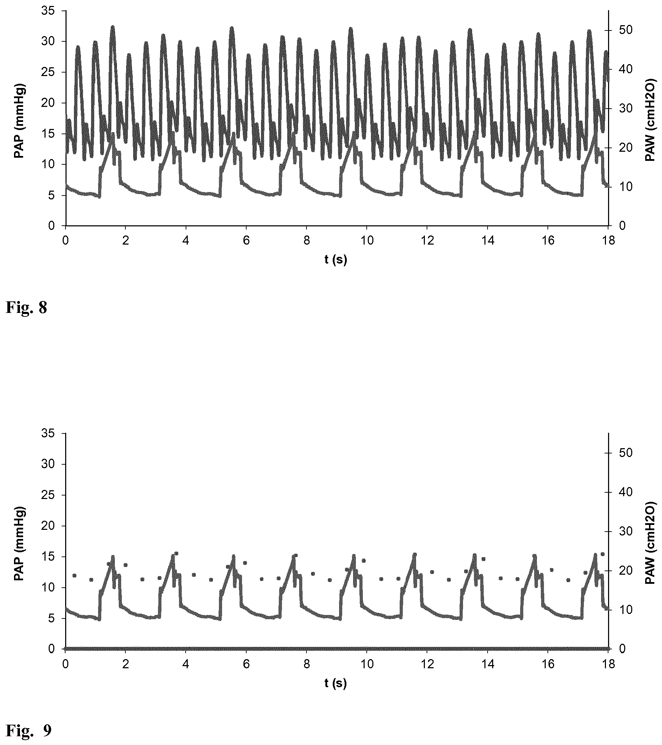

**Fig. 8****.** This figure illustrates that one of the main advantages of the use of the present invention, is that it uses all the information of the hemodynamic or cardiac signal in a way that allows performing any calculation that requires the use of analytical techniques on the signal. The calculation on all consecutive heart beats can then be rearranged according to the relative position of the cardiac cycle within the respiratory cycle.
**Fig. 9****.** This figure shows points corresponding to the value of a hemodynamic variable calculated in each of the cardiac cycles (dots). In this figure the rearrangement of the cycles has been carried out.

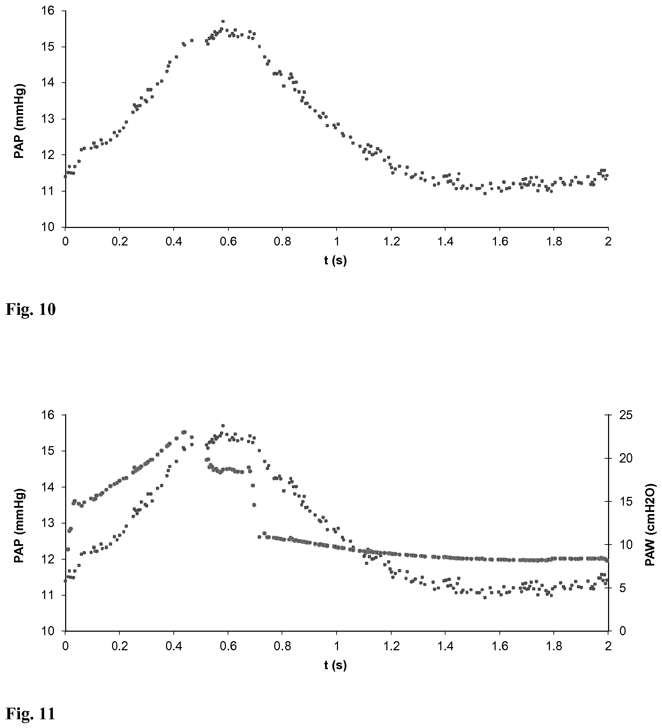

**Fig. 10****.** This figure shows how the values illustrated in figure 9 have now been reorganized according to the position they occupy within the respiratory cycle as described in step c) of the method of the present invention. Notice how now the axis of time reaches only up to 2 seconds which corresponds to the duration of the respiratory cycle.
**Fig. 11****.** This figure shows the rearrangement of cardiac cycles according to the respiratory cycle.

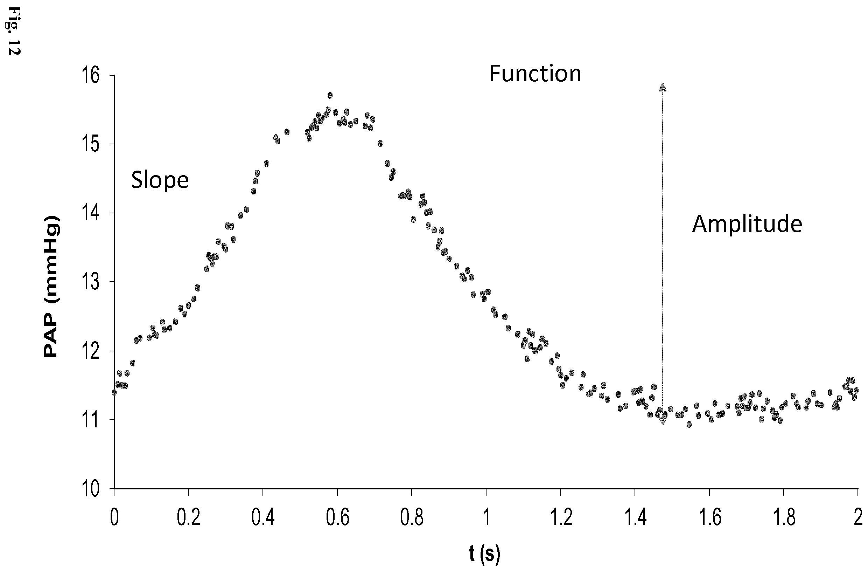

**Fig. 12****.** This figure suggests different manners of quantifying on the chart of the reordered cycles the respiratory effect on the cardiac cycle. This is because once the cycles or values of the variable analysed have been rearranged, it is easy to appreciate the component of variability that comes from breathing.

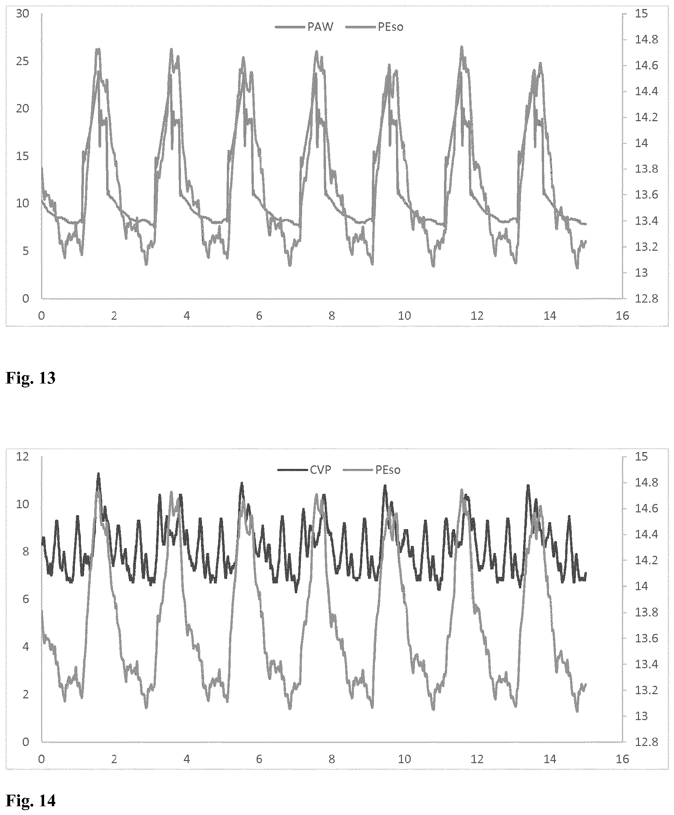

**Figs 13 to 16****.** Illustrate the implementation of the use of the present invention to determine the pleural pressure.

### Description of the invention

The methodology presented herein predicts, among other things, whether the application of MV, and in particular of high driving pressure, might lead to the development of RV failure in ARDS. The methodology of the present invention is thus preferably aim at providing protective MV strategies. In this sense, in the present invention, and by using the methodology described herein, we have demonstrated changes in PVM during breathing that are affected by the implemented driving pressure; specially, in a situation where alveoli mechanics are heterogeneous which leads to a not uniform distribution of the effect of pressure changes into the lung tissue. The results presented herein also warn about the necessity of implementing the method of the present invention, capable of providing high accuracy measurements techniques, for a better description of respiratory effects of respiration on PVM.

The use of the present invention relies on the fact that if pulmonary hemodynamic signals are reordered according to the respiratory cycle time at which the heart beat occurs, a clear respiratory modulation can be observed. In fact, such reordering, allowed us to show in the examples that PVM are cyclically affected by respiration in MV both in healthy conditions and in ARDS. Especially we demonstrated that arterial load is increased by breathing in MV and also that this increase is bigger during ARDS. These results constitute an important mechanism involved in the development of RV failure in ARDS and also help to explain the deleterious effect of high driving pressure on ARDS patients' outcomes. Also, the methodology described herein includes an almost complete beat by beat PVM evaluation accounting for static (resistance) and oscillatory (compliance) components, and wave reflection (AI) together with an estimation of RV function (DPmax).

Based on this, curves of the beat by beat calculated variables were constructed using the PA pressure foot to control the time order. Then the area under the resulting variable value-time curve was calculated and assumed to represent the total magnitude of the variable during the respiration. An asymptotic behavior was observed at the last part of respiration and the reaching value was assumed to represent the variable magnitude at exhalation. The expiratory area was calculated based on this value which was calculated as the average between the 90-95% of the respiratory time. Finally the effect of inspiration on the calculated variable was estimated as the total area minus the exhalation area. Areas were estimated by the trapezoids method after binning the signal to 0.02s intervals for smoothing purposes. Areas were normalized to the evaluated respiratory period (which allow to avoid a size effect due to the respiratory rate differences between animals) and the resulting value then represent the mean magnitude at the evaluated respiratory stage and have the same units of the corresponding variable.

It is noted that the use of the present invention is not only aimed at determining whether the application of MV, and in particular of high driving pressure, might lead to the development of PA or RV failure in ARDS; but the methodology of the present invention is capable of providing high accuracy measurements techniques, for a better description of respiratory effects of respiration on PVM, in fact, the methodology of the present invention is capable of aiding in the cardiovascular assessment of any subject in need thereof. In this sense, cardiovascular function has to be frequently monitored in patients that are mechanically ventilated, either during anesthesia and surgery or due to some disease state. The aim of such monitoring is mainly to assess the adequacy of the blood volume status of the patient and to assess cardiac function. Cardiovascular function is being widely assessed by simply measuring the blood pressure and heart rate. However, these parameters alone are notorious for being too insensitive and too unspecific to assess and follow cardiovascular changes in sick patients. There are some more advanced methods available of measuring cardiovascular function in patients who are mechanically ventilated. One of these methods is the measurement of the central venous pressure (CVP) by a catheter that is introduced through a vein into the right atrium or its vicinity. The pressure in the right atrium (CVP), however, does not always reflect the pressure in the left side of the heart, i.e., the filling pressure of the left ventricle, which is the major blood pumping mechanism. Furthermore, the CVP may be elevated due to independent failure of the right heart or some lung disease, while the left atrial pressure is, in effect, low. Finally, estimating the filling volume of a heart chamber by measuring pressure is hindered by the compliance of that chamber. These problems and also the well-known pitfalls in the interpretation of filling pressures led to the practice that in patients who suffer from circulatory failure and in whom it is crucial to diagnose the pathological mechanism of such failure for further therapy, a graded fluid loading must be performed. Such a procedure is very time-consuming and is not done very frequently.

It is thus an object of the present invention to provide a use of an apparatus for assessing cardiovascular function, preferably in ventilated patients, which does not have the disadvantages of the methods used in the prior art as discussed above. Therefore, one aspect of the invention refers to a use (from hereinafter method of the invention) for the cardiovascular assessment of a subject in need thereof according to claim 1.

As reflected above and as indicated in figure 4, it is noted that, the method of the invention requires two signals: a respiratory and a cardiac or hemodynamic signal, for obtaining the respiration cycle and the cardiac cycle of the subject. It is noted that this respiratory signal may result, but no limited to: changes in the chest volume, changes in the airway pressure, changes in the air flow or even from the cardiac or hemodynamic signal itself.

As indicated in figure 5 to practice the method of the present invention, it is deemed necessary to detect with high precision the beginning of both the respiratory cycle and the cardiac cycle; and as indicated in figure 6, once the start of the respiratory cycle is located, a new definition of the relative times of the respiratory cycle is made with respect to the same cycle. That is to say that the temporalis events begin to be considered relative to the respiratory cycle which begins to be considered as 0 once each respirable cycle begins. Taking into account that each point of the respiratory cycle can be considered in units of time whose maximum will be the duration of the respiratory cycle or in percentages or fractions with respect to each cycle, being the beginning 0 and the maximum 100% or 1. This is considered as such because the cardiac cycles will be reordered based on this relative position as indicated in step b) above. For the reordering of the cardiac cycles it is important to establish the beginning of each cardiac cycle relative to the respiratory cycle so that so that the beginning of each cardiac cycle is place and reordered relative to its position to the respiratory cycle. This is further illustrated in figure 7 wherein the numbering of the sampling points (corresponding to the hemodynamic or cardiac cycle) is carried out according to the beginning of each respiratory cycle.

As illustrated in figure 8, one of the main advantages of the method of the present invention, is that it uses all the information of the hemodynamic or cardiac signal in a way that allows performing any calculation that requires the use of analytical techniques on the signal. The calculation on all consecutive heart beats can then be rearranged according to the relative position of the cardiac cycle within the respiratory cycle. This is clearly illustrated in figure 9, which shows points corresponding to the value of a hemodynamic variable calculated in each of the cardiac cycles (dots). In this figure the rearrangement of the cycles has been carried out. This is further illustrated in figure 10, wherein it is shown how the values illustrated in figure 9 have now been reorganized according to the position they occupy within the respiratory cycle as described in step c) above. Notice how now the axis of time reaches only up to 2 seconds which corresponds to the duration of the respiratory cycle. Although these examples are performed in conditions of controlled mechanical ventilation, it is noted that the method of the present invention can also be applied in spontaneous ventilation. For this purpose, it is proposed to carry out different cycles such as the one shown in the graph but of different duration, as is the case of spontaneous ventilation.

As a further illustrative example of the present methodology, please note that figure 11 shows the rearrangement of cardiac cycles according to the respiratory cycle. Lastly, figure 12 suggests different manners of quantifying on the chart of the reordered cycles the respiratory effect on the cardiac cycle. This is because once the cycles or values of the variable analysed have been rearranged, it is easy to appreciate the component of variability that comes from breathing.

Therefore, cardiac cycle is measured from blood pressure and said blood pressure is a central venous pressure (CVP) or a peripheral venous pressure.

In another preferred embodiment of the first aspect of the invention or of any of its preferred embodiments, the respiration cycle is measured from a capnometry, a respiratory flow, an airway pressure, transthoracic electrical impedance, a plethysmogram, or a respiratory temperature.

In another preferred embodiment of the first aspect of the invention or of any of its preferred embodiments, the method further comprises a respiration determiner configured to, based on a polarity of a degree of the respiratory signal, determine whether the respiration is spontaneous respiration or artificial respiration.

In another preferred embodiment of the first aspect of the invention or of any of its preferred embodiments, the method is further characterized in that the calculating unit calculates the slope of the line of best fit of the peak or trough values of the hemodynamic response in said waveform display.

In another preferred embodiment of the first aspect of the invention or of any of its preferred embodiments, the method is further characterized in that the calculating unit calculates the area under the curve of said hemodynamic response in a certain region of the waveform display.

In another preferred embodiment of the first aspect of the invention or of any of its preferred embodiments, the method is further characterized in that the calculating unit calculates the difference between the peak or trough values of said hemodynamic response and a reference value measured during a short apnea.

In another preferred embodiment of the first aspect of the invention or of any of its preferred embodiments, the method is further characterized in that the means for measuring the hemodynamic response is adapted for measuring a parameter reflecting the left ventricular output.

On the other hand and as already stated, the present invention further relates to an apparatus by which the cardiovascular status of patients who are optionally mechanically ventilated can be assessed. The new apparatus may serve to assess the responsiveness of the patient to the administration of intravenous fluids, obviate the need for actual volume loading and the performance of invasive measurements that are currently used for such assessments.

This object is achieved by the apparatus as defined in the claims

A second example not falling under the claim refers to an apparatus suitable for cardiovascular assessment, which comprises:
1. a first sensor or means configured to detect the respiration cycle;
2. a second sensor or means configured to measure the cardiac cycle; and

one or more controllers or means configured to:
   a. detecting a starting position of at least two, preferably three, more preferably four, more preferably five, more preferably 6, 7, 8, 9, 10, 11, 12, 13, 14, 15, 16, 17, 18, 19 or at least preferably 20, consecutive or non-consecutive, respiratory cycles, and rearranging the starting position and duration of each cardiac cycle according to their position in time, with respect to the aforesaid starting position of these respiratory cycles; and
   b. building a waveform display performed by plotting a respiratory cycle as an abscissa, and by plotting each of the measurements of the cardiac cycles in relation to the time of its respective respiratory cycle's starting point obtained as described in step a) above, as an ordinate within the respiratory cycle;
wherein the cardiac and respiratory cycles are preferably synchronized and measured continuously; wherein preferably the cardiac cycles are measured from blood (or vascular) pressure and said blood (or vascular) pressure is preferably a central venous pressure (CVP) or a peripheral venous pressure; and wherein preferably each of the measurements of each cardiac cycles are selected so that there is information about the transmission or the effect of the respiration on the pleural pressure or on the volume response.

In a preferred example, the cardiac cycle is measured from an electrocardiogram, a doppler flow meter, a tonometer, a plethysmogram, blood pressure or by using the transthoracic electrical impedance.

In another example, the respiration cycle is measured from a capnometry, a respiratory flow, an airway pressure, the transthoracic electrical impedance, a plethysmogram, or a respiratory temperature.

In another example, the cardiac cycle is measured from blood pressure and said blood pressure is a central venous pressure (CVP) or a peripheral venous pressure.

In another example, the apparatus further comprises a respiration determiner configured to, based on a polarity of a degree of the respiratory signal, determine whether the respiration is spontaneous respiration or artificial respiration.

In another example, the apparatus further comprises a calculator unit configure to calculate one or more hemodynamic variables so that there is information about a particular phenomenon and how this phenomenon is affected by the respiratory cycle that predominate at the moment in which the cardiac cycle occurred, wherein the calculating unit adapted to perform analytic calculations in said waveform display. It is noted that, as a way of example, the calculator unit can be used to calculate hemodynamic variables useful in the determination or calculation of the pleural pressure (see figures 13 to 16) or any additional phenomena as indicated in any of figures 1 to 3. In this sense, the apparatus is preferably characterized in that the calculating unit calculates the slope of the line of best fit of the peak or trough values of the hemodynamic response in said waveform display. More preferably, the apparatus is characterized in that the calculating unit calculates the area under the curve of said hemodynamic response in a certain region of the waveform display. Still more preferably, the apparatus is characterized in that the calculating unit calculates the difference between the peak or trough values of said hemodynamic response and a reference value measured during a short apnea. Still more preferably, the apparatus is characterized in that the means for measuring the hemodynamic response is adapted for measuring a parameter reflecting the left ventricular output.

The apparatus of the example may further comprise a respirator and/or a monitor. The monitor will preferably be equipped with special software that after applying the method of the invention may further measure the changes in any hemodynamic parameter (e.g., blood pressure, plethysmographic signal, Doppler echo, etc.).

The apparatus of the example can be used in all mechanically ventilated patients or in non-mechanically ventilated patients, preferably the apparatus may be use in mechanically ventilated patients to determine whether the application of MV, and in particular of high driving pressure, might lead to the development of PA or RV failure in ARDS. The apparatus may thus be use for providing protective MV strategies. The apparatus of the example can also serve as a basic diagnostic test apparatus for the determination of volume responsiveness, which is very high during frank hypovolemia and very low or negative during congestive heart failure and/or volume overload. It may be used in anesthetized patients and in all other patients that are mechanically ventilated by any ventilatory mode. With the apparatus of the present example, it is hence possible to easily measure the cardiovascular status by medical equipment normally used in any mechanically ventilated patient without the need of additional complicated, costly and difficult-to-use equipment.

A third example also not falling under the scope of the claims refers to a computer program for implementing a method according to the first aspect or to any of its preferred embodiments, when the computer program is executed by a computer or processor.

Finally, we would like to summarize the technical effects achieved by the present invention. In this sense, figure 13 of the present application, shows a simultaneous recording of airway (PAW) and esophageal pressure (PEso). PEso is the clinical standard for pleural pressure estimation. Being the graph an example of a passive ventilated individual (pig) the PEso reflects the transmission of PAW to the pleural space. In this regard, it is well-accepted that PEso represents the pressure in such space. From the figure, it can be observed that the fluctuations in the PEso are related with fluctuations occurring in the respiratory system. Also a smaller fluctuation at a higher frequency related with direct transmission of the heart beat to the PEso can be seen (red circle). These fluctuations occur for the spatial proximity of the esophageal balloon placed to measure PEso with the heart.

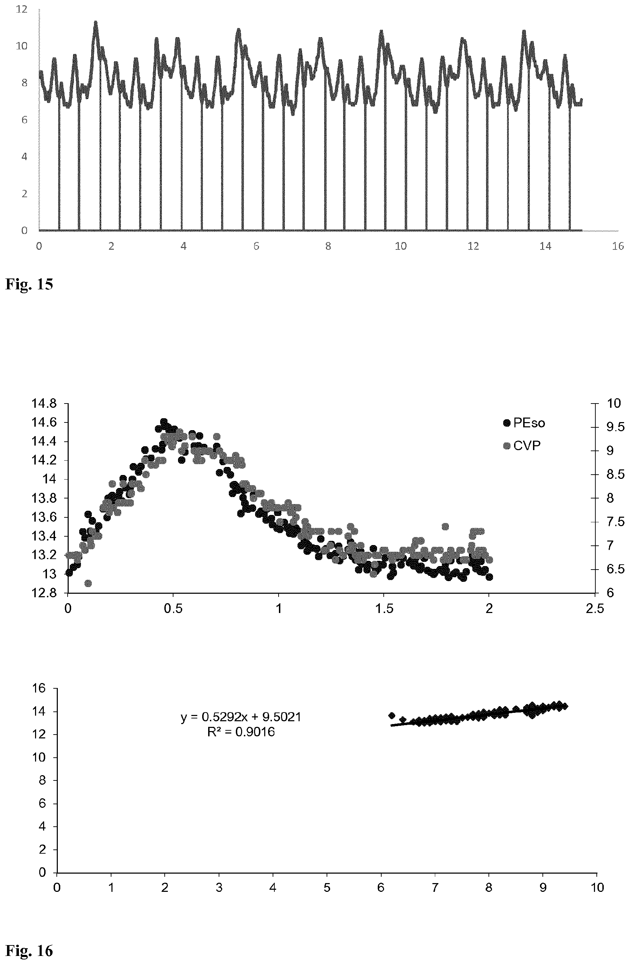

On the other hand in figure 14 simultaneous recording of central venous pressure and (CVP) and PEso is shown. Besides of the characteristic waveform (higher frequency fluctuations) of the CVP representing different heart events (atrial contraction, ventricle contraction, heart filling, between others) a lower frequency fluctuation related with breathing can be observed (highest amplitude fluctuation in the PEso). It is thus reasonable to assume that a particular point of the CVP waveform will represent better the transmission of pleural pressure change into the vascular system (figure 15). Such a point can be for example detected every other heart cycle.

As it can be observed in figure 14 and 15, due to the differences in heart and respiratory frequencies, the analysis of the chosen point in the CVP waveform only during the period of one breath cannot be enough to describe the entire effect respiration fluctuation and its transmission to the vascular system. This is the reason why reordering heart cycle according to its relative position in the respiratory cycle would increase the possibility of describing the respiratory fluctuation transmission into the cardiovascular system. In figure 16 it can be seen that if only data from CVP waveform from one respiratory cycle period were analysed, the number of points for the plot were the result of the ratio between respiratory and heart period. In the particular example (figure 14, 15 and 16 come from the same situation) these number of points were between 3 and 4. However increasing the studied period of time and reordering CVP waveform points according to its position in the respiratory cycle significantly increases the ability to describe the waveform describing the respiratory fluctuation. The adequacy of the chosen point to represent the PEso (and in consequence the pleural pressure) can be corroborated in the resulting correlation (Figure 16) coming from the chosen CVP points and PEso measured at the same time (figure 16).

Such description of the waveform representing the transmission or the effect of the respiration on the cardiovascular system will represent the pleural pressure if a particular point representative of this is chosen as shown in the example. However the respiratory effect on the cardiovascular system can be related with other factors and used with other purposes as the fluid response prediction, ventricle function and arterial load. For this the proposed method can be implemented in several other hemodynamic signals as an arterial pressure signal and different representative points or hemodynamic variables can be analysed.

The following examples merely illustrate the present invention but do not limit the same.

### Examples

### Materials and Methods

This study was conducted at the Hedenstierna laboratory, Uppsala University, Sweden and approved by the Institutional Animal Care and Use Committee. Six pigs (*sus scrofa domesticus*, Norwegian-Yorkshire breed) (31.7 ± 5.4 kg) were studied.

### Anesthesia and instrumentation

After inducing anesthesia, animals were tracheotomized and connected to mechanical ventilation (MV) (Servo-i. Maquet Critical Care, Solna, Sweden). Intravenous anesthesia was then maintained using a combination of ketamine, midazolam and fentanyl, adding rocuronium for muscle relaxation after adequate anesthesia was ascertained by lack of reaction to painful stimulation between the front hooves. Baseline ventilatory settings consisted of positive end expiratory pressure (PEEP) 8cmH₂O, tidal volume 6ml/kg, inspiratory/expiratory ratio (I:E) 1:2, FIO₂ 1 and respiratory rate adjusted to keep an end-tidal CO₂ around 45mmHg in volume-controlled ventilation. A 4-lumen central catheter and a pulmonary artery (PA) catheter (Edwards, Irving, CA, USA) were inserted through the right internal jugular vein. A small left lateral thoracotomy was performed to expose the PA trunk around which an ultrasonic flow probe (Confidence Flowprobes, PAU series, Transonic, Ithaca, NY, USA) was placed as proximal to the RV as possible. A micro-tip pressure transducer (Mikro-Tip, SPR-340, Millar, Houston, TX, USA) was inserted directly into the PA at the level of the ultrasonic probe. Thereafter, the thorax was carefully closed layer by layer.

Monitoring also included electrocardiogram, femoral artery pressure and airway flow and pressure measured through a pneumotacograph attached to the endotracheal tube and a NICO (Philips, Wallingford, CT, USA).

After instrumentation animals were submitted to a lung volume history homogenization maneuver by changing to pressure controlled ventilation mode with PEEP 20cmH₂O, inspiratory driving pressure 20cmH₂O, respiratory rate 20 bpm and I:E 1:1 for 30 seconds. Baseline measurements were obtained 15 min thereafter.

### ARDS model

Saline (30ml/kg) lung lavages were performed until a PaO₂/FIO₂ < 200mmHg was reached at PEEP 8cmH₂O and FIO₂ 1.0. Animals were then submitted to two hours of injurious ventilation (PEEP 0cmH₂O and inspiratory driving pressure 35cmH₂O). Once the model was established, ventilatory parameters were changed to baseline and a new set of measurements was obtained after stabilization for one hour. Intravenous boluses of normal saline (5ml/kg) were administered if mean systemic arterial pressure was < 60mmHg during the model creation. No vasoactive drugs were used at the time of evaluation.

Two to three continuous minute stable signal period was selected at baseline and ARDS avoiding extra-systoles and other undesired variability sources.

### Signal acquisition and processing

The flow probe was attached to a Perivascular Flow Module (TS420, Transonic, Ithaca, NY, USA) and the pressure probe to a PowerLab Data Acquisition System (PowerLab 16/35, Adinstruments, Dunedin, New Zealand). Both systems were connected to a MP-150 Biopac Data Acquisition System (MP-150WSW, Biopac Systems, Inc., Goleta, CA, USA) for simultaneous data acquisition together with ECG, airway pressure and flow and other physiological signals at 200 or 1000 hz (then resampled to 200 hz). Signals were processed using the Acknowledge software (ACK100W, Biopac Systems, Inc., Goleta CA, USA) and LabChart 8 (Adinstruments, Dunedin, New Zealand). For continuous analysis, signal were smoothed applying a Savitzky-Golay 2nd order filter with an 11 points window which was also applied for continuous first and second derivative calculus when necessary. All signal analysis were performed using customized routines programmed in Excel (Microsoft Corporation, Redmond, WA, USA) and Matlab (Mathworks, Inc, Natick MA, USA).

### Correction of the airway pressure change effect on pulmonary artery pressure

Airway pressure transmission affect, PA pressure measurement (Figure 1a). This is particularly important when the measurements are performed during active breathing due to the airway pressure cyclic changes. In this invention we proposed a method for decreasing this effect by tracking the PA pressure diastolic change across the respiratory cycle. This was based on the similitude between the airway and diastolic pressure change during the MV respiratory cycle (figure 1b). Also, from a physiologic point of view, late on diastole most of the pressure exerted by the cardiac contraction has gone and the vessel could be affected by the transmission of an external pressure easier. To track the airway transmitted effect the minimum pressure at the start of each PA pressure cycle was determined. Then cardiac beats were reordered according to the time at which this minimum pressure occurs during the respiratory cycle (for this inhalation start was defined as 0.01s before airway flow higher than 51/min was reached which was always above the signal noise). After reordering, the evident close relation between airway and diastolic pressure is observed (figure 1c). A smoothing splines algorithm was applied to estimate missing signal at some time points by interpolation allowing slight signal smoothing but preserving main curve shape. For this, the curve was repeated avoiding distortion at start and end assuming that this signal is continuous and cyclically invariable during the evaluated period. Then signal was scaled to its minimum value and this new function was applied to all the evaluated time period at intervals defined by respiratory cycle assuming such invariability. Finally this signal was subtracted from measured PA pressure (during the same period from which the correction curve was found).

### Beat by beat Pulmonary Vascular Mechanics evaluation

Flow and PA pressure (corrected signal) foots were detected and an algorithm starting at each new pressure and flow cycle was applied. Beat by beat detection of the required criteria for PVM evaluation was performed based on waveform analysis. For calculation of variables requiring flow and pressure information beats were synchronized using the electrocardiogram as fiduciary signal. We calculated:
- **Stroke volume (SV):** Area under the flow-time curve in systole.
- **Mean pressure:** Total pressure-time curve divided by the cardiac period.
- **Pulse pressure:** Systolic minus foot pressure.
- **Cardiac (pressure) period:** Estimated as the foot to foot duration of the pressure cycle.
- **Arterial compliance:** SV/Pulse pressures.
- **Resistance:** mean pressure/mean flow.
- **Augmentation index (AI):** Augmented pressure/Pulse pressure. Augmented pressure was calculated as systolic pressure minus the pressure at the second zero crossing (negative to positive) of the pressure second derivative (inflection point).
- **Inflection point time:** Time at the inflection point (normalized to cardiac period).
   Augmentation index and inflection point time accounts for the effect of reflected waves on PA pressure. The higher the AI and the shorter the inflection point time the bigger the reflected wave effect and the worse its effect on PVM.
- **Effective arterial elastance (Eaeff):** mean pressure/SV. It represents a global measure of arterial load accounting for the pulsatile nature of circulation⁶.
- **Dpmax:** First maximum of pulmonary artery first derivative. It has been described as directly related to ventricle contractility. It was informed also as normalized to stroke volume (Dpmax/SV).

### Estimation of the evaluated variables performance across the respiratory cycle

If pulmonary hemodynamic signals are reordered according to the respiratory cycle time at which the heart beat occurs a clear respiratory modulation can be observed. Based on this, curves of the beat by beat calculated variables were constructed using the PA pressure foot to control the time order. Then the area under the resulting variable value-time curve was calculated and assumed to represent the total magnitude of the variable during the respiration. An asymptotic behavior was observed at the last part of respiration and the reaching value was assumed to represent the variable magnitude at exhalation. The expiratory area was calculated based on this value which was calculated as the average between the 90-95% of the respiratory time. Finally the effect of inspiration on the calculated variable was estimated as the total area minus the exhalation area. Areas were estimated by the trapezoids method after binning the signal to 0.02s intervals for smoothing purposes. Areas were normalized to the evaluated respiratory period (which allow to avoid a size effect due to the respiratory rate differences between animals) and the resulting value then represent the mean magnitude at the evaluated respiratory stage and have the same units of the corresponding variable.

### Other physiological measurements

NICO pressure and flow signal acquired at 100hz were ensemble averaged and respiratory mechanics was evaluated as usually described. For a better description of the tidal respiratory mechanics stress index was calculated following previously described criteria. Arterial and mixed venous blood gas variables were also measured and shunt was estimated using the Berggren equation⁷.

### Statistical analysis

Data were expressed as mean (standard deviation) unless otherwise specified. Normality was assessed using the Shapiro-Wilk test. Paired t-test and Wilcoxon's test (for non-normally distributed data) were used for comparing measurements in Baseline versus ARDS. Significance was considered at p <0.05. Significance of the inspiratory curve was interpreted as change in the evaluated variable caused by respiration. Statistical analysis was performed with Microsoft excel 2003 (Microsoft Corporation, Redmond, WA, USA) and Stata v15 (StataCorp, College Station, TX, USA).

### Results

All of the animals had simultaneous recording of the respiratory and PA signals. The heart to respiratory rate ratio during the studied time period was 2.9±0.6 in baseline and 3.0±0.5 in ARDS (p=0.819).

Respiratory variables are presented in table 1.

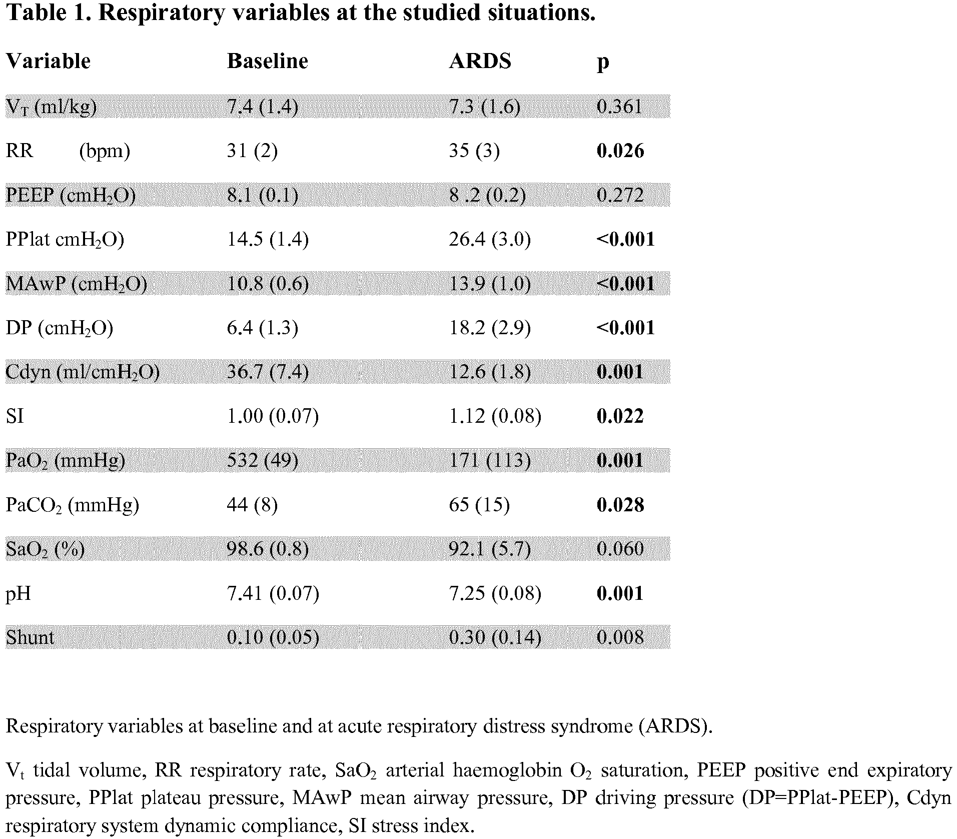

ARDS resulted in significant worsening of respiratory mechanics and gas exchange. ARDS leaded to cyclic overdystension as demonstrated by an increase in stress index to values above 1.1.

Respiration decreased SV, mean and pulse PA pressure, and compliance in both baseline and ARDS and Dpmax just in baseline, and increased PA resistance and Eaeff. When compared to baseline, ARDS decreased SV and PA compliance and increased PA mean pressure, Eaeff, Dpmax and Dpmax/VS when evaluated during total breathing cycle and at expiration. The inspiratory decrease in PA mean and pulse pressure and the increase in Eaeff (figure 3) was larger in ARDS than in baseline. No differences could be demonstrated for wave reflection phenomena, although a trend to a larger magnitude (AI) and a faster arrival (Inflection point time) of the reflected wave was observed during ARDS. Interestingly pressure cycles period did not change during respiration. Data shown in table 2.

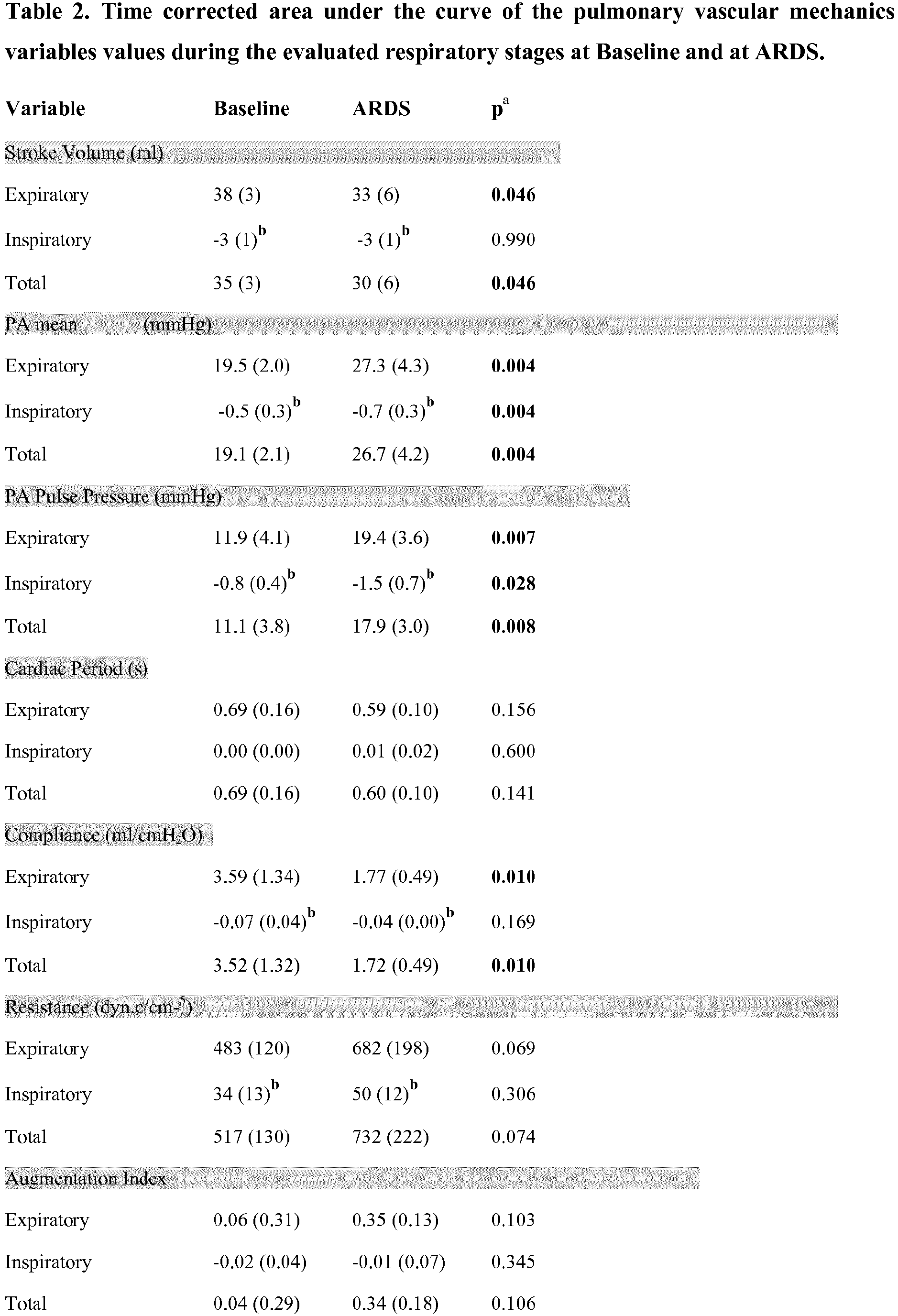

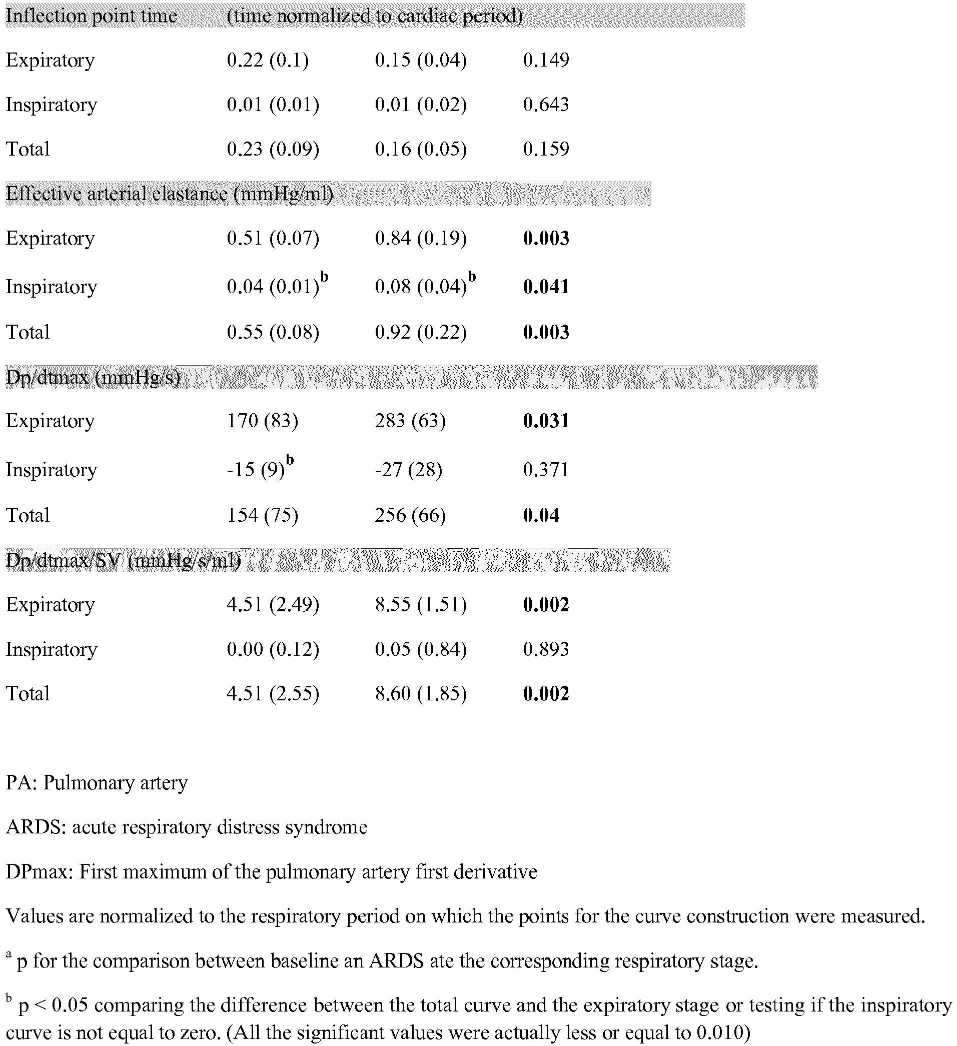

### Acknowledgments

This study was funded by the Swedish Heart and Lung foundation and the Swedish Research Council (K2015-99X-22731-01-4) and by the European Critical Care Research Network (ECCRN) and the European Society of Intensive Care Medicine, Basic Sciences Award 2012.

AS received fund from Fundación Conchita Rábago, Madrid Spain for his maintenance during the development of this study.

AS is M+Visión COFUND Advanced Fellow and has received funding from *Consejería de Educatión, Juventud y Deporte of the Comunidad de Madrid* and the People Programme (Marie Curie Actions) of the *European Union's Seventh Framework Programme* (FP7/2007-2013) under Research Executive Agency grant agreement n° 291820.

## Claims

1. Use of an apparatus suitable for cardiovascular assessment, for the determination of a transmission or an effect of the respiration on the pleural pressure or on the volume response, wherein the apparatus comprises:
a first sensor configured to detect respiration cycles;
a second sensor configured to measure cardiac cycles; and
one or more controllers configured to:
a. detect a starting position of at least two, preferably three, more preferably four, more preferably five, more preferably 6, 7, 8, 9, 10, 11, 12, 13, 14, 15, 16, 17, 18, 19 or at least preferably 20, consecutive or non-consecutive, respiratory cycles, and rearranging the starting position and duration of each cardiac cycle according to their position in time, with respect to the aforesaid starting position of these respiratory cycles; and
b. building a waveform display performed by plotting a respiratory cycle as an abscissa, and by plotting each of the measurements of the cardiac cycles in relation to the time of its respective respiratory cycle's starting point obtained as described in step a) above, as an ordinate within the respiratory cycle;
wherein the cardiac and respiratory cycles are preferably synchronized and measured continuously and wherein the cardiac cycles are measured from central venous pressure (CVP) or a peripheral venous pressure;
wherein each of the measurements of each cardiac cycles are selected so that there is information about the transmission or the effect of the respiration on the pleural pressure or on the volume response; and
wherein the apparatus further comprises a calculator unit which is adapted to perform analytic calculations from said waveform display to calculate one or more hemodynamic variable.

2. The use of the apparatus according to claim 1, wherein such use is for the determination of the pleural pressure and wherein each of the measurements of each cardiac cycles are selected so that there is information about the transmission or the effect of the respiration on the pleural pressure.

3. The use of an apparatus according to claim 1 or 2, wherein the respiration cycles are measured from a capnometry, a respiratory flow, an airway pressure, the transthoracic electrical impedance, a plethysmogram, or a respiratory temperature.

4. The use of an apparatus according to any of claims 1 to 3, further comprising a respiration determiner configured to, based on a polarity of a degree of the respiratory signal, determine whether the respiration is spontaneous respiration or artificial respiration.

## Patentansprüche

1. Verwendung einer Vorrichtung, welche zur kardiovaskulären Beurteilung, zur Bestimmung einer Übertragung oder eines Effekts der Atmung auf den Pleuraldruck oder auf die Volumenantwort geeignet ist, wobei die Vorrichtung Folgendes umfasst:
einen ersten Sensor, welcher dazu ausgebildet ist, Atmungszyklen zu erfassen;
einen zweiten Sensor, welcher dazu ausgebildet ist, Herzzyklen zu messen; und
eine oder mehrere Steuerungen, welche dazu ausgebildet sind:
a. eine Anfangsstellung von mindestens zwei, vorzugsweise drei, weiter vorzugsweise vier, weiter vorzugsweise fünf, weiter vorzugsweise 6, 7, 8, 9, 10, 11, 12, 13, 14, 15, 16, 17, 18, 19 oder mindestens vorzugsweise 20, aufeinanderfolgenden oder nicht aufeinanderfolgenden, Atmungszyklen zu erfassen, und die Anfangsstellung und die Länge jedes Herzzyklus gemäß deren zeitlichen Lage, in Bezug auf die zuvor erwähnten Anfangsstellung dieser Atmungszyklen umzuordnen; und
b. eine Wellenformanzeige zu erstellen, welche durch das Darstellen eines Atmungszyklus als Abszisse, und durch das Darstellen jeder der Messungen der Herzzyklen im Verhältnis zum Zeitpunkt dessen jeweiligen Anfangspunktes des Atmungszyklus, welcher wie zuvor in Schritt a) beschrieben wurde erhalten wurde, als Ordinate innerhalb des Atmungszyklus durchgeführt wird;
wobei die Herz- und Atmungszyklen vorzugsweise synchronisiert und durchgängig gemessen werden und wobei die Herzzyklen aus dem zentralen Venendruck (CVP) oder einem peripheren Venendruck gemessen werden;
wobei jede der Messungen jedes Herzzyklus so ausgewählt werden, dass es Information über die Übertragung oder den Effekt der Atmung auf den Pleuraldruck oder auf die Volumenantwort gibt, und
wobei die Vorrichtung zusätzlich eine Recheneinheit umfasst, welche dazu angepasst ist, analytische Berechnungen aus der genannten Wellenformanzeige durchzuführen, um eine oder mehrere hämodynamische Variablen zu berechnen.

2. Verwendung der Vorrichtung nach Anspruch 1, wobei solche Verwendung zur Bestimmung des Pleuraldrucks ist und wobei jede der Messungen jedes Herzzyklus so ausgewählt werden, dass es Information über die Übertragung oder den Effekt der Atmung auf den Pleuraldruck gibt.

3. Verwendung einer Vorrichtung nach Anspruch 1 oder 2, wobei die Atmungszyklen aus einer Kapnometrie, einem Atemstrom, einem Atemwegdruck, der transthorakalen elektrischen Impedanz, einem Plethysmogramm oder einer Atemtemperatur gemessen werden.

4. Verwendung einer Vorrichtung nach einem der Ansprüche 1 bis 3, zusätzlich umfassend eine Atmungsbestimmungseinrichtung, welche dazu ausgebildet ist, basierend auf einer Polarität eines Grads des Atmungssignals, zu bestimmen, ob die Atmung eine Spontanatmung oder eine künstliche Atmung ist.

## Revendications

1. Utilisation d'un appareil d'évaluation cardiovasculaire, pour la détermination d'une transmission ou d'un effet de la respiration sur la pression pleurale ou sur la réponse en volume, dans laquelle l'appareil comprend :
un premier capteur configuré pour détecter des cycles de respiration ;
un second capteur configuré pour mesurer des cycles cardiaques ; et
un ou plus contrôleurs configurés pour :
a. détecter une position de début d'au moins deux, préférablement trois, plus préférablement quatre, plus préférablement cinq, plus préférablement 6, 7, 8, 9, 10, 11, 12, 13, 14, 15, 16, 17, 18, 19 ou au moins préférablement 20, cycles respiratoires, consécutifs ou non consécutifs, et réorganiser la position de début et la durée de chaque cycle cardiaque selon leur position dans le temps, par rapport à ladite position de début de ces cycles respiratoires ; et
b. développer un affichage de forme d'onde réalisé en traçant un cycle respiratoire comme une abscisse, et en traçant chacune des mesures des cycles cardiaques par rapport au temps du point de début de son cycle respiratoire respectif, obtenu tel que décrit dans l'étape a) ci-dessus, comme une ordonnée dans le cycle respiratoire ;
dans laquelle les cycles cardiaques et respiratoires sont préférablement synchronisés et mesurés continuellement et dans laquelle les cycles cardiaques sont mesurés à partir de la pression veineuse centrale (PVC) ou une pression veineuse périphérique ;
dans laquelle chacune des mesures de chaque cycle cardiaque est sélectionnée de sorte qu'il y a des informations sur la transmission ou l'effet de la respiration sur la pression pleurale ou sur la réponse en volume ; et
dans laquelle l'appareil comprend en outre une unité de calcul qui est adaptée pour réaliser des calculs analytiques à partir dudit affichage de forme d'onde pour calculer une ou plusieurs variables hémodynamiques.

2. Utilisation de l'appareil selon la revendication 1, dans laquelle une telle utilisation est pour la détermination de la pression pleurale et dans laquelle chacune des mesures de chaque cycle cardiaque est sélectionnée de sorte qu'il y a des informations sur la transmission ou l'effet de la respiration sur la pression pleurale.

3. Utilisation d'un appareil selon la revendication 1 ou 2, dans laquelle les cycles de respiration sont mesurés à partir d'une capnométrie, un flux respiratoire, une pression des voies respiratoires, l'impédance électrique transthoracique, un pléthysmogramme ou une température respiratoire.

4. Utilisation d'un appareil selon l'une quelconque des revendications 1 à 3, comprenant en outre un déterminant de respiration configuré pour déterminer, basé sur une polarité d'un degré du signal respiratoire, si la respiration est de la respiration spontanée ou de la respiration artificielle.
